# EUROPÄISCHE PATENTANMELDUNG

(11) **EP 3 354 198 A1**
(43) Veröffentlichungstag der Anmeldung: **01.08.2018**
(21) Anmeldenummer: 18151795.4
(22) Anmeldetag: 16.01.2018
(51) Int. Cl.: A61B 5/117, A61B 90/90, A61B 5/00, A61B 5/1172

(54) **VERFAHREN UND VORRICHTUNG ZUR ERFASSUNG UND VERARBEITUNG VON MEDIZINISCHEN DATEN**

(30) Priorität: 27.01.2017 DE 102017000892
(71) Anmelder: seca ag, 4153 Reinach BL 1 (CH)
(72) Erfinder: LEUNER, Rüdiger, 21107 Hamburg (DE); THOMAS, Eric, 22926 Ahrensburg (DE); WIETZKE, Timo, 20259 Hamburg (DE)
(74) Vertreter: Klickow & Wetzel PartGmbB

(57) **Zusammenfassung**

Das Verfahren und die Vorrichtung dienen zur Erfassung und Verarbeitung von medizinischen Daten. Von einer Verarbeitungseinrichtung wird über mindestens eine Datenerfassungseinheit eine Identifizierungsinformation erfasst. Die Identifizierungsinformation wird über eine Kommunikationseinheit an eine Zentraleinheit übertragen. Die Verarbeitungseinrichtung wird einer Messeinrichtung zugeordnet und als Identifizierungsinformation wird mindestens eine Patientenidentifizierung erfasst.

## Beschreibung

Die Erfindung betrifft ein Verfahren zur Erfassung und Verarbeitung von medizinischen Daten, bei dem von einer Verarbeitungseinrichtung über mindestens eine Datenerfassungseinheit mindestens eine Identifizierungsinformation erfasst und über eine Kommunikationseinheit an eine Zentraleinheit übertragen wird.

Die Erfindung betrifft darüber hinaus eine Vorrichtung zur Erfassung und Verarbeitung von medizinischen Daten, bei der mindestens eine Funktionskomponente in einem Gehäuse angeordnet ist und bei der mindestens eine der Funktionskomponenten als eine Kommunikationseinheit und mindestens eine weitere Funktionskomponente als ein Dateneingang ausgebildet ist.

Derartige Verfahren und Vorrichtungen sind bereits in unterschiedlichen Ausführungsformen im Bereich der Medizintechnik bekannt. Eine Verwendung erfolgt beispielsweise im Bereich von Arztpraxen, Krankenhäusern oder Krankenwagen. Ein Problem bei der Erfassung und Verarbeitung von medizinischen Daten besteht darin, dass für eine sichere Diagnose und die Auswahl geeigneter therapeutischer Maßnahmen eine verwechslungssichere Zuordnung der erfassten medizinischen Daten zum jeweiligen Patienten erfolgen muss. Darüber hinaus treten Probleme häufig dadurch auf, dass nicht bekannt oder zumindest nicht nachvollziehbar ist, mit welchem medizintechnischen Gerät die entsprechenden Daten erfasst wurden und welches medizinische Personal die entsprechenden Tätigkeiten durchgeführt hat.

Aufgabe der vorliegenden Erfindung ist es daher, ein Verfahren der einleitend genannten Art derart zu verbessern, dass eine exakte Zuordnung von Messdaten zu einem bestimmten Patienten unterstützt wird.

Diese Aufgabe wird erfindungsgemäß dadurch gelöst, dass die Verarbeitungseinrichtung einer Messeinrichtung zugeordnet wird und dass als Identifizierungsinformation mindestens eine Patientenidentifizierung erfasst wird.

Weitere Aufgabe der vorliegenden Erfindung ist es, eine Vorrichtung der einleitend genannten Art derart zu konstruieren, dass eine exakte Zuordnung von medizinischen Messdaten zum jeweiligen Patienten unterstützt wird.

Diese Aufgabe wird erfindungsgemäß dadurch gelöst, dass der Dateneingang zur Verbindung mit einer Datenerfassungseinrichtung ausgebildet ist.

Durch die Verwendung des erfindungsgemäßen Verfahrens und der erfindungsgemäßen Vorrichtung ist es nicht mehr erforderlich, transportable Computer oder ähnliche Einrichtungen im Bereich des Patienten zu verwenden und hierbei durch zeitlich aufeinander folgende Verwendungen bei unterschiedlichen Patienten insbesondere im Bereich von Krankenhäusern die Verbreitung von Infektionen zu riskieren.

Erfindungsgemäß ist insbesondere daran gedacht, die Vorrichtung fest an einem bestimmten Gerät anzuordnen und hierdurch eine verwechslungssichere Zuordnung zu unterstützen.

Der Begriff der festen Verbindung beinhaltet hierbei auch eine zwar grundsätzlich trennbare Anordnung, die während des bestimmungsgemäßen Gebrauches typischerweise jedoch nicht verändert wird.

Eine Funktionskontrolle durch Bedienpersonal wird dadurch unterstützt, dass im Bereich des Gehäuses mindestens eine Statusanzeige angeordnet ist.

Eine visuell leicht erkennbare Funktionsüberwachung wird dadurch realisiert, dass mindestens eine der Statusanzeigen als LED ausgebildet ist.

Für einen Datenaustausch ist vorgesehen, dass im Bereich des Gehäuses mindestens ein Netzwerkanschluss angeordnet ist.

Gemäß einer typischen Ausführungsform ist daran gedacht, dass die Datenerfassungseinrichtung als ein Barcode-Scanner ausgebildet ist.

Gemäß einer weiteren Ausführungsform ist es auch möglich, dass die Datenerfassungseinrichtung als ein Iris-Scanner ausgebildet ist.

Ebenfalls ist es möglich, dass die Datenerfassungseinrichtung als ein Fingerabdruck-Scanner ausgebildet ist.

Die Anordnung der Vorrichtung im Bereich eines medizintechnischen Gerätes wird dadurch unterstützt, dass das Gehäuse einen Halterungsbereich zur Kopplung mit einer Halterungseinrichtung aufweist.

Eine äußerst einfache Positionierung des Gehäuses wird dadurch unterstütz, dass der Halterungsbereich mindestens teilweise als eine seitliche Vertiefung des Gehäuses ausgebildet ist.

Eine sowohl funktionelle als auch gut handhabbare und visuell ansprechende Gestaltung wird dadurch erreicht, dass das Gehäuse mindestens bereichsweise in einer Querschnittfläche eine ovalartige Begrenzung aufweist.

Verschiedene Ausführungsbeispiele und Ausgestaltungen der Erfindung sind in den nachfolgenden Figuren abgebildet. Es zeigen
- Fig. 1: eine perspektivische außenseitige Ansicht einer Vorrichtung zur Erfassung und Verarbeitung von medizinischen Daten, die eine oval artig begrenzte Gehäusekontur aufweist und bei der eine Halterung durch eine federnde Klammer realisierbar ist,
- Fig. 2: eine Seitenansicht der Vorrichtung gemäß Blickrichtung II. in Figur 1,
- Fig. 3: eine Ansicht gemäß Blickrichtung III. in Figur 1,
- Fig. 4: eine weitere Draufsicht auf die Vorrichtung gemäß Figur 1,
- Fig. 5: eine Ansicht gemäß Blickrichtung V. in Figur 1,
- Fig. 6: eine Darstellung der Vorrichtung gemäß Figur 1 in einem teilweise auseinandergenommenen Zustand,
- Fig. 7: eine teilweise geschnittene Darstellung der Vorrichtung gemäß Figur 2 in einem unteren Bereich,
- Fig. 8: eine Anordnung der Vorrichtung gemäß Figur 1 im Bereich einer Waage,
- Fig. 9: eine gegenüber Figur 8 abgewandelte Verwendung im Bereich einer anderen Waage,
- Fig. 10: eine Darstellung ähnlich zu Figur 2 bei einer von der Halterungsklammer getrennten Vorrichtung und
- Fig. 11: zeigt eine weitere Darstellung der Vorrichtung gemäß Figur 1 nach einer Trennung der Vorrichtung von der Halterungsklammer.

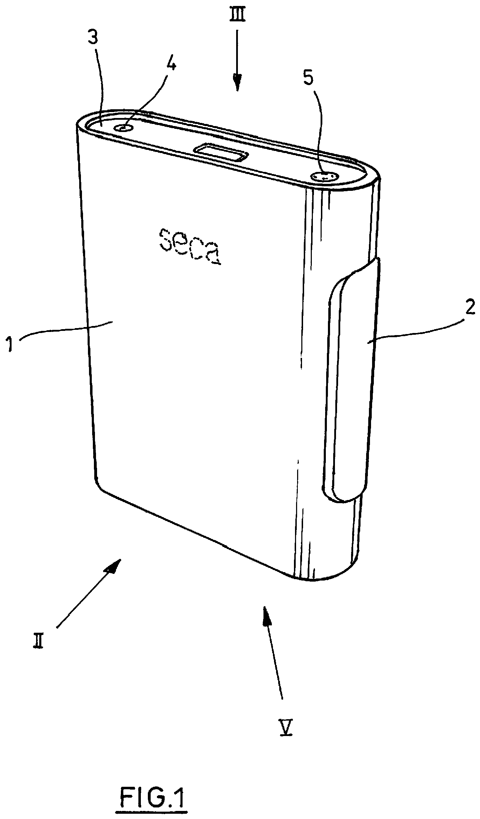

Gemäß der Ausführungsform in Figur 1 weist die Vorrichtung ein Gehäuse (1) auf, das von einer Halterungseinrichtung (2) positionierbar ist. Die Halterungseinrichtung (2) kann federnd und klammerartig ausgebildet sein. Im Bereich einer Oberseite (3) sind am Gehäuse (1) Statusanzeigen (4, 5) angeordnet. Die Statusanzeigen (4, 5) können beispielsweise als LEDs ausgeführt sein. Insbesondere ist daran gedacht, die Statusanzeigen (4, 5) jeweils als grün oder rot leuchtende Anzeigen auszubilden, wobei grün einen störungsfreien und rot einen störungsbehafteten Funktionszustand anzeigt. Die Statusanzeigen (4, 5) können sich beispielsweise auf den Betriebsstatus und/oder den Verbindungsstatus beziehen.

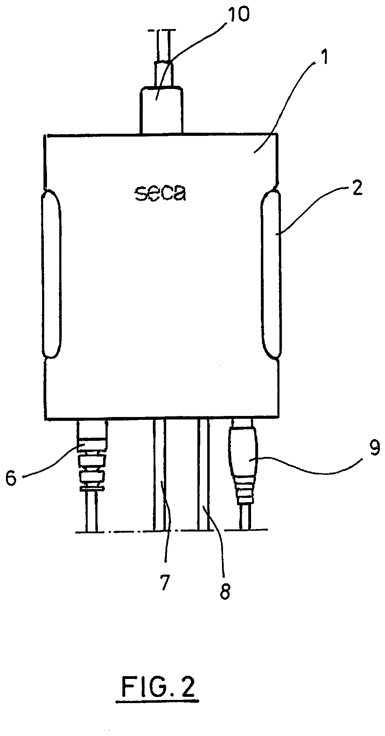

Gemäß der Ausführungsform in Figur 2 besitzt das Gehäuse (1) einen LAN-Anschluss (6), ISIS-Bus-Anschlüsse (7, 8) sowie einen Netzanschluss (9). Darüber hinaus ist ein USB-Anschluss (10) vorgesehen.

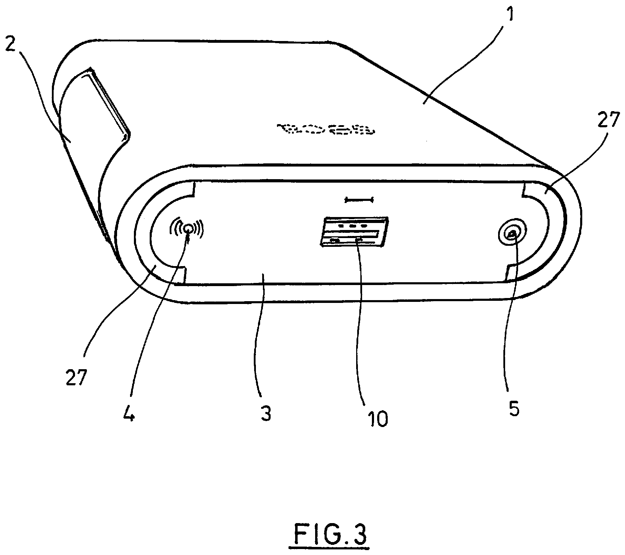

Die perspektivische Ansicht gemäß Figur 3 veranschaulicht die Anordnung des USB-Anschlusses (10) sowie der Statusanzeigen (4, 5) im Bereich der Oberseite (3) des Gehäuses (1). Zusätzlich sind hier auch noch Statusanzeigen (27) dargestellt, die jeweils eine halbkreisartige Gestaltung aufweisen.

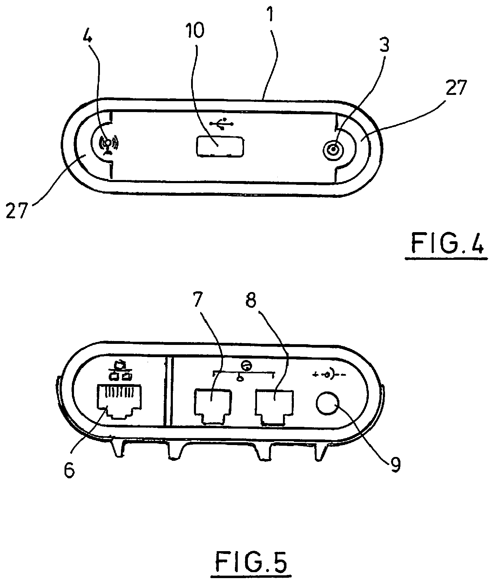

Die gleichen Funktionselemente sind auch in der Draufsicht gemäß Figur 4 zu erkennen.

Gemäß der unterseitigen Ansicht in Figur 5 sind der LAN-Anschluss (6), die beiden ISIS-Bus-Anschlüsse (7, 8) sowie der Netzanschluss (9) zu erkennen. Alternativ oder ergänzend kann auch ein WLAN-Modul verwendet werden.

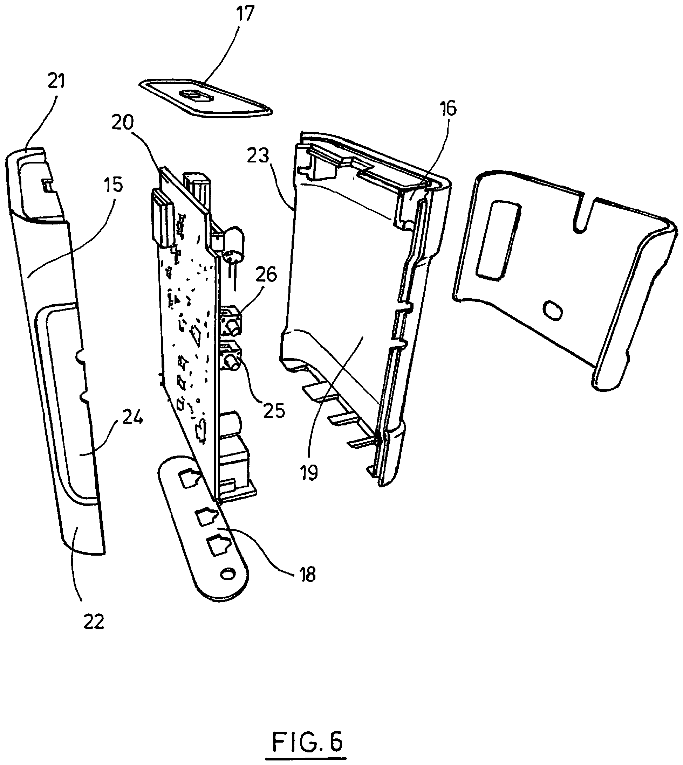

Figur 6 zeigt ähnlich zu einer Explosionsdarstellung einen teilweise auseinander genommenen Zustand der Vorrichtung. Es ist zu erkennen, dass das Gehäuse (1) aus einer Oberschale (15) und einer Unterschale (16) besteht. Darüber hinaus sind im Bereich der Oberseite (3) ein Deckel (17) und im Bereich einer Unterseite ein Boden (18) verwendet. In einem vom Gehäuse (1) umschlossenen Raum (19) ist eine Platine (20) angeordnet, die die wesentlichen elektrischen und elektronischen Funktionsbaugruppen haltert.

Zu erkennen ist in Figur 6 auch, dass die klammerartige Haltungseinrichtung (2) bevorzugt einteilig ausgebildet ist. Typischerweise ist hier an eine Ausbildung aus einem Kunststoff gedacht.

Zur Unterstützung einer Halterung des Gehäuses (1) durch die Halterungseinrichtung (2) besitzt das Gehäuse (1) im Bereich von Seitenflanken (21, 22) jeweils muldenartige langgestreckte Vertiefungen (23, 24). Die Gestaltung der Vertiefungen (23, 24) ist an die Gestaltung der klammerartigen Struktur der Haltungseinrichtung (2) angepasst.

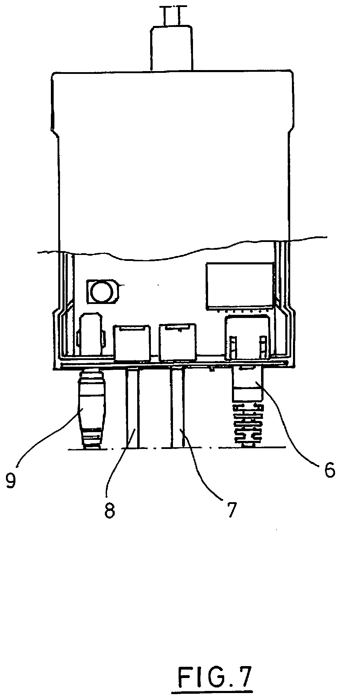

Figur 7 zeigt in einer teilweise geschnittenen Darstellung die Zuordnung der Anschlüsse (7, 8, 9, 10) zum Gehäuse (1) sowie zur Platine (20). Die Anschlüsse (6, 7, 8, 9, 10) können gemäß einer bevorzugten Ausführungsform buchsenartig ausgebildet sein und dienen zur Aufnahme von mit Steckern versehenen Anschlusskabeln.

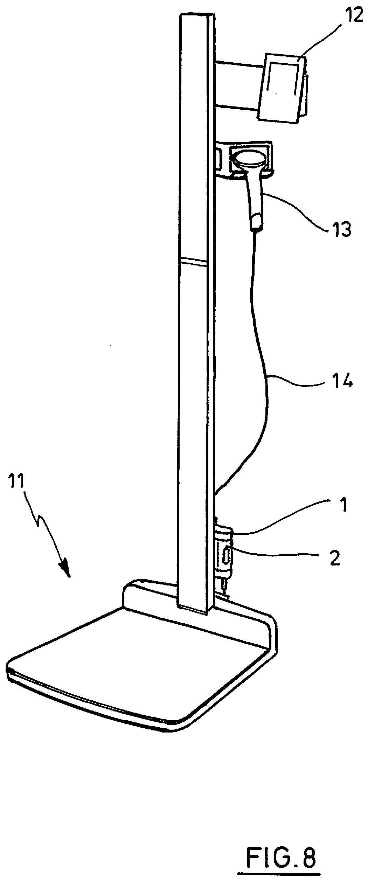

Die perspektivische Darstellung gemäß Figur 8 veranschaulicht die Anordnung der Vorrichtung im Bereich einer Waage (11). Die Waage ist mit einem Display (12) ausgestattet und zur gerätetechnischen Unterstützung einer Erfassung von Patientenidentifikationen wird eine Messeinrichtung (13) verwendet. Beim dargestellten Ausführungsbeispiel ist die Messeinrichtung (13) als ein Handscanner ausgebildet, der über ein Kabel (14) mit der Vorrichtung verbindbar ist. Die Messeinrichtung (13) kann beispielsweise als ein Barcode-Scanner, ein Iris-Scanner, ein Nahfeld-Scanner (NFR) oder ein Fingerabdruck-Scanner ausgebildet sein.

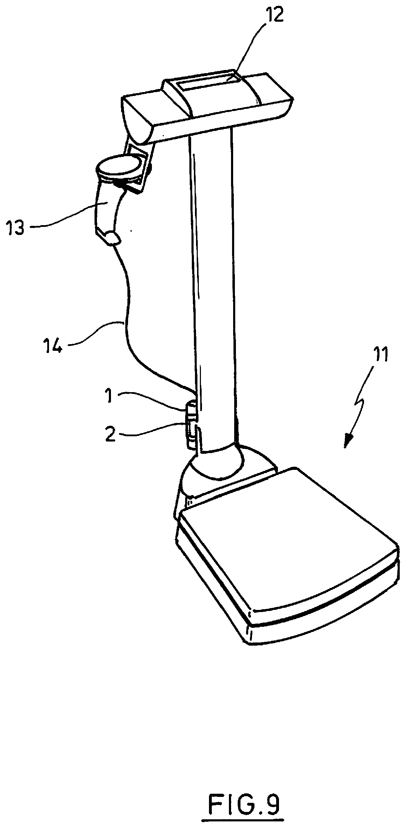

Gemäß der Ausführungsform in Figur 9 erfolgt eine Anordnung im Bereich einer anderen Waage (11).

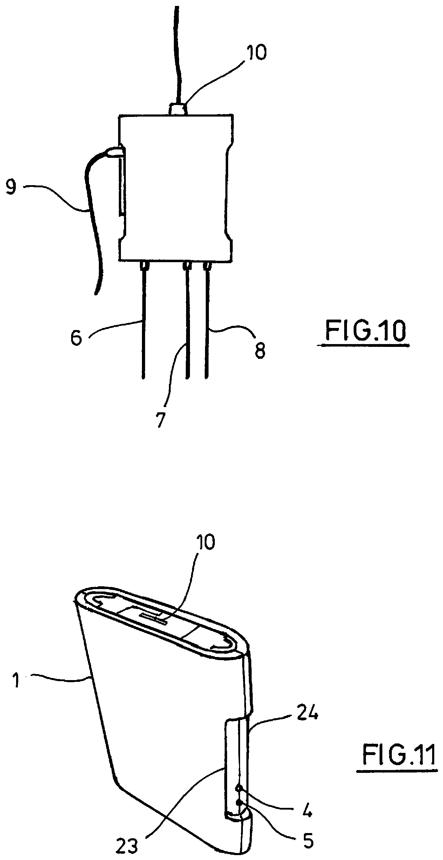

Gemäß der Ausführungsform in Figur 10 erfolgt ein Anschluss von mit Steckern versehen Kabeln ähnlich wie gemäß der Darstellung in Figur 2. Zusätzlich ist hier in einem seitlichen Bereich noch der Anschluss einer weiteren Verbindung zur Datenübertragung.

Figur 11 zeigt das Gehäuse (1) in einem von der Halterungseinrichtung (2) getrennten Betriebszustand. Zu erkennen ist, dass hier im Bereich der Vertiefung (24) zwei Tastschalter (25, 26) angeordnet sind. Nach einem Zusammenfügen des Gehäuses (1) und der Halterungseinrichtung (2) sind die Tastschalter (25, 26) abgedeckt und hierdurch gegen eine unbeabsichtigte Bedienung gesichert.

Grundsätzlich kann die Vorrichtung unter Verwendung einer geeigneten Halterungseinrichtung (2) beliebig im Bereich eines medizintechnischen Gerätes angeordnet werden. Bevorzugt ist daran gedacht, die Oberschale (15) und die Unterschale (16) durch Rasteinrichtungen zusammenzufügen und hierdurch die Verwendung sichtbarer Schrauben zu vermeiden. Zur Unterstützung einer korrekten Zuordnung der jeweiligen Stecker zu den Anschlüssen (6, 7, 8, 9, 10) ist insbesondere daran gedacht, geeignete Symbole direkt an den jeweiligen Stecker-Interfaces anzuordnen. Hinsichtlich der Gestaltung des Gehäuses (1) ist insbesondere daran gedacht, zwei im Wesentlichen parallel zueinander verlaufende Seitenflächen durch kreisabschnittförmige Flankenbereiche miteinander zu verbinden. Auftretende Fasen können markant gestaltet werden. Beispielsweise ist es möglich, das Gehäuse (1) aus einem hellen Kunststoff und die Halterungseinrichtung (2) aus einem dunklen Kunststoff zu fertigen. Hierdurch entsteht ein visuell ansprechender optischer Kontrast.

## Patentansprüche

1. Verfahren zur Erfassung und Verarbeitung von medizinischen Daten, bei dem von einer Verarbeitungseinrichtung über mindestens eine Datenerfassungseinheit mindestens eine Identifizierungsinformation erfasst und über eine Kommunikationseinheit an eine Zentraleinheit übertragen wird, **dadurch gekennzeichnet, dass** die Verarbeitungseinrichtung einer Messeinrichtung zugeordnet wird und dass als Identifizierungsinformation mindestens eine Patientenidentifizierung erfasst wird.

2. Vorrichtung zur Erfassung und Verarbeitung von medizinischen Daten, bei der mindestens eine Funktionskomponente in einem Gehäuse angeordnet ist und bei der mindestens eine der Funktionskomponenten als eine Kommunikationseinheit und mindestens eine weitere Funktionskomponente als ein Dateneingang ausgebildet ist, **dadurch gekennzeichnet, dass** der Dateneingang zur Verbindung mit einer Datenerfassungseinrichtung (13) ausgebildet ist.

3. Vorrichtung nach Anspruch 2, **dadurch gekennzeichnet, dass** im Bereich des Gehäuses (1) mindestens eine Statusanzeige (4, 5,27) angeordnet ist.

4. Vorrichtung nach Anspruch 3, **dadurch gekennzeichnet, dass** mindestens eine der Statusanzeigen (4, 5,27) als LED ausgebildet ist.

5. Vorrichtung nach einem der Ansprüche 2 bis 4, **dadurch gekennzeichnet, dass** im Bereich des Gehäuses (1) mindestens ein Netzwerkanschluss angeordnet ist.

6. Vorrichtung nach einem der Ansprüche 2 bis 5, **dadurch gekennzeichnet, dass** die Datenerfassungseinrichtung (13) als ein Barcode-Scanner ausgebildet ist.

7. Vorrichtung nach einem der Ansprüche 2 bis 6, **dadurch gekennzeichnet, dass** die Datenerfassungseinrichtung (13) als ein Iris-Scanner ausgebildet ist.

8. Vorrichtung nach einem der Ansprüche 2 bis 7, **dadurch gekennzeichnet, dass** die Datenerfassungseinrichtung (13) als ein Fingerabdruck-Scanner ausgebildet ist.

9. Vorrichtung nach einem der Ansprüche 2 bis 8, **dadurch gekennzeichnet, dass** das Gehäuse (1) einen Halterungsbereich zur Kopplung mit einer Halterungseinrichtung (2) aufweist.

10. Vorrichtung nach Anspruch 9, **dadurch gekennzeichnet, dass** der Halterungsbereich mindestens teilweise als eine seitliche Vertiefung des Gehäuses (1) ausgebildet ist.

11. Vorrichtung nach einem der Ansprüche 2 bis 10, **dadurch gekennzeichnet, dass** das Gehäuse (1) mindestens bereichsweise in einer Querschnittfläche eine ovalartige Begrenzung aufweist.

12. Vorrichtung nach einem der Ansprüche 2 bis 4, **dadurch gekennzeichnet, dass** im Bereich des Gehäuses (1) mindestens ein WLAN-Modul angeordnet ist.

13. Vorrichtung nach einem der Ansprüche 2 bis 6, **dadurch gekennzeichnet, dass** die Datenerfassungseinrichtung (13) als ein Nahfeld-Scanner (NFC-Scanner) ausgebildet ist.
